# EUROPEAN PATENT SPECIFICATION

(11) **EP 3 631 747 B1**
(45) Date of publication and mention of the grant of the patent: **07.07.2021**
(21) Application number: 18734946.9
(22) Date of filing: 31.05.2018
(51) Int. Cl.: G06Q 50/30, G16H 50/30

(54) **METHOD AND SYSTEM FOR THE EVALUATION OF THE RISK OF AORTIC RUPTURE OR DISSECTION IN AN INDIVIDUAL WITH AN ASCENDING THORACIC AORTIC ANEURYSM**
VERFAHREN UND SYSTEM ZUR BEWERTUNG DES RISIKOS DER AORTENRUPTUR ODER -DISSEKTION IN EINEM INDIVIDUUM MIT EINEM AUFSTEIGENDEN THORAKALEN AORTENANEURYSMA
PROCÉDÉ ET SYSTÈME D'ÉVALUATION DU RISQUE DE RUPTURE OU DE DISSECTION AORTIQUE CHEZ UN INDIVIDU PRÉSENTANT UN ANÉVRISME DE L'AORTE THORACIQUE ASCENDANTE

(30) Priority: 31.05.2017 IT 201700059572
(43) Date of publication of application: 08.04.2020
(73) Proprietor: Fondazione Ri.MED, 90133 Palermo (IT); Istituto Mediterraneo Per I Trapianti E Terapie Ad Alta Specializzazione S.r.l - I.S.M.E.T.T. S.r.l, 90133 Palermo (IT)
(72) Inventor: PASTA, Salvatore, 90133 Palermo (IT); SCARDULLA, Cesare, 90133 Palermo (IT)
(74) Representative: Capasso, Olga
(86) International application number: PCT/IB2018/053882
(87) International publication number: WO 2018/220573

(56) References cited:
- WO-A1-2017/075268
- WO-A2-2014/004889
- US-A1- 2015 164 342

## Description

### TECHNICAL FIELD

The present invention relates to a method for calculating the risk of aortic rupture or dissection in an individual with an ascending thoracic aortic aneurysm (ATAA). Furthermore, the invention relates to a system for applying said method as an instrument for the clinical decision-making relating to the operation of an ascending thoracic aortic aneurysm.

### STATE OF THE ART

An aneurysm represents an abnormal dilation or a permanent swelling of an artery portion and is caused by a weakening of the wall of the blood vessel. An aneurysm of the ascending thoracic aorta (ATAA) involves the ascending part of the aorta, which is the largest artery in the human body and serves as a channel for the blood flow, which circulates towards the distal parts of the body. The most common cause of an ATAA is a condition known as "cystic medial necrosis" whose etiology is partly unknown. The elastic fibres, which make up the tunica media of the aorta can degenerate as a person ages, and this condition makes the aorta more prone to dilation. Risk factors predisposing the formation of an ATAA include severe hypertension, smoking, atherosclerosis, genetic syndromes of the connective tissue, such as Marfan and, above all, bicuspid aortic valve (BAV) (Coady MA, et al. : Natural history, pathogenesis, and etiology of thoracic aortic aneurysms and dissections. Cardiol Clin 1999;17:615-35; vii). An ATAA is widely recognized as a clinically silent condition (asymptomatic) until fatal complications arise, such as rupture or dissection (Elefteriades JA: Natural history of thoracic aortic aneurysms: Indications for surgery, and surgical versus nonsurgical risks. Ann Thorac Surg 2002;74:S1877-S80). A diagnosis is normally made by means of a diagnostic test (ultrasound, computed tomography or magnetic resonance) after an individual manifests chest pain.

The bicuspid aortic valve (BAV) typically comprises two cusps (or edges) of different sizes, and differs from the normal-morphological aortic valve, which has three cusps (TAV). Even though a BAV is a congenital malformation, which affects only 1-2% (Ward C: Clinical significance of the bicuspid aortic valve. Heart 2000;83: 81-85) of the world's population, such pathological condition represents the commonest factor predisposing the formation of an ATAA and is the main cause of mortality and morbidity in relation to all other congenital heart defects. Patients affected by BAV have a 9-times higher risk of developing complications related to ATAA compared to subjects with TAV, and an 80% higher risk of developing a dilation of the ascending aorta (Rampoldi V, et al.: Simple risk models to predict surgical mortality in acute type A aortic dissection: the International Registry of Acute Aortic Dissection score. Ann Thorac Surg 2007; 83:55-61). Based on the incidence of the BAV pathology and Italian population (approximately 60,000,000, source ISTAT), it is estimated that there are between about 0.6 and 1,200,000 individuals with a BAV in Italy, and most of these show a high probability of developing complications related to the pathological valve or ATAA at the age of 70 years old.

If an ATAA is not treated appropriately by means of surgery, or monitored over time, the aneurysm can determine complications, such as the rupture or dissection of the blood vessel consequently resulting in an individual's death. At present, for patients with an ATAA, close monitoring of the size of the aneurysm by computed tomography (CT) and magnetic resonance (MR) is the only way available to determine when it is necessary to intervene surgically to avoid fatal complications (Elefteriades JA, Farkas EA: Thoracic aortic aneurysm clinically pertinent controversies and uncertainties. J Am Coll Cardiol 2010;55:841-57). In fact, physical examinations are generally unable to detect the presence of an ATAA since this disease is asymptomatic. The current clinical criterion is based on the measurement of the maximum diameter of the aorta, which can be estimated by means of a radiological diagnostic examination. The diagnostics for routine images to measure the size of the aorta over time is generally carried out every 6-12 months after the initial diagnosis. However, in such period of time, constant growth of a portion of the aorta might occur, as well as complications of the arterial vessel. European guidelines recommend that an ATAA must be replaced or, if necessary, repaired with a prosthesis, when the diameter of the aorta is about 5.0 centimetres, and in the case of risk factors, or an increase in the diameter of the aorta of more than 0.2 centimetres a year.

This approach is justified by the annual rate of aortic dissection, which increases gradually as the size of the aorta grows (Tsai TT, et al.: Long-term survival in patients presenting with type A acute aortic dissection: insights from the International Registry of Acute Aortic Dissection (IRAD). Circulation 2006;114:1350-6). In patients with BAV or genetic syndromes (for example, Marfan) a lower intervention threshold of 4.5 centimetres is recommended. However, this approach may be considered highly aggressive because patients with ATAA are generally old (the average age of patients at the time of diagnosis is 60 years old) and this significantly increases the risks related to the surgical operation.

It has been shown that, even with an aggressive approach, preventive surgery with an aortic diameter ≤4.5cm is unable to prevent 40% of dissections of an ATAA (Pape LA, et al.: Aortic diameter ≥ 5.5 cm is not a good predictor of type A aortic dissection - Observations from the international registry of acute aortic dissection (IRAD). Circulation 2007;116:1120-7.). Patients with an aortic diameter who have reached a maximum diameter of 6.0 centimetres have the following annual rates of fatal events: rupture (3.6%), dissection (3.7%) and death (10.8%). This suggests that the current clinical criterion based on the maximum aortic diameter has a low prognostic value of the risk related to the disease. Therefore, the clinical dilemma is to determine a reliable cut-off for an aneurysm, which can be monitored (but with a risk related to non-intervention) and the aneurysm, which must be operated on (but with a risk related to surgical treatment).

Given the growing percentage of old people in industrialized countries, the increased frequency of chronic hypertension, and an improvement in the quality of life, the clinical management of patients with an ATAA represents an increasingly arduous challenge for the health system. People are living longer and enjoying a healthier lifestyle in parts of the world where the quality of life has improved. An ageing society can influence the financial impact that an ATAA has on the health system and, consequently, the capacity of each State to provide resources for older citizens. Other factors, such as blood pressure and hypertension, increase the probability of individuals developing an aneurysm. Furthermore, people and society's general well-being has increased over the past ten years and these conditions are improving rapidly. Therefore, new technology is required for a better clinical management of ATAAs, including personalized strategies for individuals or groups of individuals in order to optimize the financial-health management of the disease. Technology of this kind should be based on stratification criteria of the clinical risk, which take into account patients' characteristics in order to rationalize diagnostic monitoring and reduce the financial cost on the health system. The therapeutic approach should be based on sound physical principles rather than on a single epidemiological criterion, which applies to everyone (in other words, "one-size-fits-all"), such as the current approach of the maximum aortic diameter.

US 2015/0164342 A1 discloses a method for calculating a risk index of aortic diseases. No considerations concerning non-coding RNA biomarkers are therein disclosed.

It is an object of the present invention to overcome the aforesaid drawbacks of the known methods and provide a method and system, which is more effective and practical.

### DESCRIPTION OF THE INVENTION

Therefore, a method and system for calculating a risk index of aortic rupture or dissection in an individual with ascending thoracic aortic aneurysm according to the independent claims are presented herein.

The method according to the present invention comprises the steps of obtaining a first data set related to the individual's clinical and/or demographic characteristics, obtaining a second data set related to a biological sample of the individual's biochemical characteristics and obtaining a third data set related to the morphological and functional characteristics of the aorta. Furthermore, the method comprises the step of processing the third data set to obtain a fourth data set by computational modelling, and integrating the first data set, the second data set, the third data set and the fourth data set in a prediction model to obtain a risk index of aortic rupture or dissection.

The method according to the present invention is characterized in that the second data set comprises expression values of at least one non-coding RNA biomarker chosen from the group comprising: miR-16, miR-9 miR-101, miR-143, miR-19, miR-21, miR-29, and miR-423-5p.

This method can represent a clinical decision support system (CDSS). By combining information relating to the individual's demographic data, personalized computational modelling of the aneurysm and biomarkers obtained from the individual's biological sample, it is possible to keep track of the changes in an individual's condition, day by day, with the aim of providing a highly accurate projection of the risk of complications of an aneurysm, as well as a better allocation of the clinical resources. The end result of the method is a risk index, which informs the clinic in good time of the probability of complications of the aneurysm, so that the doctor is better informed and can thus determine the patient's therapy more effectively. Such instrument is also important for less skilled doctors, and in more uncertain clinical cases, as a guiding instrument for the choice of the therapy to be followed. In particular, the risk index can assume a value from "0" (absence of risk) to "1" (maximum risk). Such interval serves to indicate the ATAA risk level, which is obtained subject to "training" compared to a control population, as described below.

Once an individual has been diagnosed with an ascending thoracic aortic aneurysm (ATAA), various clinical and demographic data is collected and radiological diagnostic and laboratory medicine examinations are carried out. This information is entered into the CDSS as input variables for the decision-making support to create a patient's profile.

The term "demographic characteristics" refers, for example, to the individual's age, sex, race and weight.

The term "clinical characteristics" refers to all of the individual's information concerning their familial predisposition to developing an ATAA. Examples of clinical data include blood pressure, potential high blood pressure, consumption of cigarettes, use of drugs, such as beta-blocker and ACE-inhibitors/sartans, previous history of surgical operations or chest deformations, presence/absence of coronary disease, the presence of diabetes, collagen diseases, kidney failure, genetic syndromes, such as Marfan syndrome, and congenital malformation of the aortic valve, in other words, BAV.

The term "biochemical characteristics" refers to all of the biochemical laboratory data, which can be obtained from the individual's biological sample, for example a blood sample.

The term "morphological and functional characteristics" refers to data relating to the measurement of the size of the aorta and to the evaluation of the functionality of the aortic valve. Examples of morphological data include the measurements of the diameter of the aorta to the annulus, the aortic root, the sino-tubular junction and the ascending aorta. Examples of functional data include echocardiographic parameters, like the orifice of the aortic valve, the transaortic flow, aortic insufficiency and/or stenosis and the transaortic pressure gradient.

The use of biomarkers obtained from biochemical analyses of laboratory medicine can be useful in assessing damage to the tissue of the aortic wall caused by the progression of the aneurysm. The biomarker expression values are integrated into an ATAA decision-making by means of the method according to the present invention. This makes the method a personalized approach, which is more effective for making a diagnosis, but, above all, managing and monitoring the aneurysm compared to the traditional methods based on guidelines related to epidemiological information. In particular, the computational method used in the method according to the present invention integrates structural characteristics of the behaviour of the aortic vessel, specific of an aneurysm and the valve thereof, in a fluid-structure model, which are unique compared to the theoretical assumptions of other types of computational analyses.

In order to determine an epigenetic profile of the aneurysm, the biomarker can be represented by at least one from non-coding RNA molecules (miRNA), metalloproteinase of the extracellular matrix (MMP) and tissue inhibitors (TIMP). Such epigenetic screening can be carried out using traditional laboratory medicine methods.

Specifically, the non-coding RNA can be chosen from the group comprising: miR-16, miR-9 miR-101, miR-143, miR-19, miR-21, miR-29, and miR-423-5p. In a preferred embodiment, the second data set (22) further comprises expression values of at least one non-coding RNA biomarker chosen from the group comprising: miR-133a, miR-155, miR-320a, miR-34a (MI0001251), miR-34a (MI0000268) .

In particular, the first set of miRNAs (miR-16, miR-9 miR-101, miR-143, miR-19, miR-21, miR-29, and miR-423-5p) allows the presence of the aneurysm to be identified, and miR-133a, miR-155, miR-320a, miR-34a (MI0001251), miR-34a (MI0000268) allow an ATAA of an individual with TAV to be distinguished from one with a BAV.
Additionally, the metalloproteinase of the extracellular matrix can be MMP-9 and the tissue inhibitor TIMP-1.

Thanks to the combination of epigenetic data and computational calculations, it is possible to obtain a personalized stratification for the individual, thus guaranteeing a more rigorous decision-making in order to distinguish a "benign" aneurysm from a "malign" aneurysm more reliably and more accurately. In fact, such approach is based on physical principles rather than on a doctor's experience or merely on clinical evidence, for example the maximum aortic diameter of an ATAA.

In particular, computational modelling offers the unique advantage of providing specific patient information on the hemodynamic functionality of the vessel compared to that obtainable from the current clinical criterion. On the contrary, the epigenetic data provides indications on the deterioration of the wall of the ATAA and therefore on the genetic mechanisms related to the onset of an aneurysm. However, clinical evidence shows that there are multiple morphological phenotypes of BAV and dilations of the aortic vessel, with manifestations, which differ from person to person. This heterogeneity of the pathology of an ATAA is the reason why the diagnostic/prognostic value provided by the biomarkers or the computational model alone is limited and which, on the contrary, needs to integrate the information, which can be obtained from various disciplines to consider the contribution of each of the pathogenetic factors (for example, genetics and hemodynamics) present in an individual. Such approach undoubtedly results in a personalized therapy and allows the clinical decision-making to be rationalized.

Furthermore, the biomarker can be represented by at least one from C-reactive protein (access number:NP_000558; version:NP_000558.2), creatine kinase (access number:NP_001814; version:NP_001814.2), Nt-proBNP (access number:NP_002512; version:NP_002512.1), cardiac troponin I (access number:NP_000354; version:NP_000354.4), from the advanced glycation end product AGE (access number:P51606; version:P51606.2) and corresponding receptor RAGE (access number:ACF47656; version:ACF47656.1), from the transforming growth factor beta TGF-beta (access number: NP_000651; version: NP_000651.3), D-dimer (access number: 2Q9I_F; version: 2Q9I_F) and interleukin 6 (access number:NP_000591; version:NP_000591.1). Specifically, these are biomarkers of cardiovascular lesions, inflammation and fibrosis, as well as metabolomics.

Among these, TGF-beta is a soluble cytokine, which affects vascular remodelling. An alteration thereof may contribute to the onset of Marfan syndrome, or dilation of the aortic root or dissection. Similarly, the combination AGE-RAGE may play an important role in vascular dysfunction and prove useful as a potential biomarker in ATAA. In fact, the expression of AGE-RAGE is significantly altered in patients with a thoracic aneurysm. D-dimer is associated with the fragmentation of fibrin in coagulopathy and is currently used to identify pulmonary embolisms. It is worth noting that the use of only one of these biomarkers probably has a low prognostic significance on the risk of complications of an ATAA. Therefore, to improve the predictive capacity of the method according to the present invention, it is possible to aggregate a set of different biomarkers.

In one embodiment of the invention, the third data set can comprise morphological data after a virtual reconstruction of the anatomy of the individual's aorta by means of a diagnostic imaging method.

In particular, a computerized tomography (CT), magnetic resonance (MR or MRI) or ultrasound can be used to reconstruct the virtual anatomy of the individual's aorta in 3D.
A CT is preferable to an MR for measuring the size of the aorta as it offers a higher resolution. An echocardiography cannot be used due to the limited spatial resolution. However, an echo-transesophageal analysis image can be used for the purposes of the present invention. According to the method described in the present invention, one of these three imaging techniques of the aneurysmatic vessel is needed (in other words, CT, MR or echo-transesophageal).

Furthermore, the fourth data set can comprise hemodynamic and structural parameters of the aorta estimated by means of a numerical simulation and wherein said hemodynamic and structural parameters are integrated in a bidirectional fluid-structure model, which allows the behavior of the movement of the blood (fluid) to be described inside the walls of the vessel (solid).

Thanks to this fluid-structure model, it is possible to estimate the hemodynamics in a completely non-invasive way, in other words, the blood flow in the vessel, as well as the structural behaviour of the aneurysm and thus identify the areas of the aortic wall, which are most likely to develop complications. Hemodynamic variables and structural parameters are extrapolated from the numerical simulation and subsequently used in the predictive analytical model. These parameters are available without having to expose the individual to the risk of invasive procedures or to further clinical tests other than those recommended, for example, by the doctor.

In particular, the method can further comprise processing of the results of the numerical simulation to display the hemodynamic and structural parameters superimposing them on the virtual reconstruction of the anatomy of the aorta and extrapolating said parameters in different anatomical positions of the aorta.

In this way, it is possible to identify the points on the virtual reconstruction where the aorta may present problems.

According to one embodiment of the invention, the hemodynamic and structural parameters can comprise at least the blood pressure, shear stress, intramural stress and the helicoidal flow index.

In this way, it is possible to use the computational parameters to identify highly stressed areas of the aortic wall and consequently at a higher risk of developing complications and thus more in need of attention by the doctor.

Additionally, the fourth data set can comprise information relating to a deformation of the aorta and a time variation of said deformation obtained by applying a time tracking algorithm.

Starting from the CT, MR or ultrasound data, a time tracking algorithm for the wall of the vessel can be used to evaluate the distribution of the deformations of the aneurysmatic wall. Like the computational modelling, the area with more deformations is at a higher risk of complications. The information relating to the deformations and time variation of the same is entered as input variables to implement the predictive model together with the aforesaid variables (demographic and clinical data, biomarkers, hemodynamic and structural parameters) and obtain an overall risk index.

In one embodiment of the invention, the predictive model can be automatically refined after an iterative process.

Advantageously, the method according to the present invention is based on automatic learning of all of the variables entered, to perfect the predictive capacity of the risk of complications, subject to "training" of the predictive algorithm itself on a previously observed control population, composed of both healthy individuals, in other words, individuals not affected by an ATAA and individuals with an ATAA.

In particular, to refine the predictive capacity of this method, the variables are entered iteratively into the statistic model until there is a convergence towards a common root so as to refine the predictive capacity of the same. The higher the number of cases entered into the model, the better the predictive capacity of the same is.

The method can also comprise an assessment of the weight of each datum belonging to the first, second, third of fourth data set on the risk index.

In this way, it is possible to determine the prognostic significance of the variables gathered for the individual and classify the case in question in the control population (for healthy individuals) or among people with ATAA.
For the purposes of evaluating the risk index, the sum of the variables will have a weight of "1" and the impact of each single variable will vary from person to person. For example, the morphological characteristic will have a greater impact on one individual, while the biochemical characteristics from the markers might have a greater impact on another.

The decision system for calculating the risk of aortic rupture or dissection of an individual with an ascending thoracic aortic aneurysm according to the present invention comprises first means for obtaining a first data set linked to the clinical and/or demographic characteristics of the individual, second means for obtaining a second data set linked to the biochemical characteristics of a biological sample of the individual, third means for obtaining a third data set linked to the morphological and functional characteristics of the aorta and fourth means for obtaining a fourth data set obtained from processing of the third data set by computational modelling. Furthermore, the system comprises a computer having a data interface for receiving the first data set, the second data set, the third data set and the fourth data set as input data. In particular, the computer comprises a processor for processing said data and issuing a risk index of aortic rupture or dissection as output data integrating the first, the second, the third and the fourth data set in a predictive model, wherein the second data set comprises expression values of at least one non-coding RNA biomarker chosen from the group comprising: miR-16, miR-9 miR-101, miR-143, miR-19, miR-21, miR-29, and miR-423-5p.
Preferably, the second data set (22) comprises further expression values of at least one non-coding RNA biomarker chosen from the group comprising: for miR-133a, miR-155, miR-320a, miR-34a (MI0001251), miR-34a (MI0000268).
This system is configured for the application of the decision method for calculating the risk of aortic rupture or dissection of an individual with an ascending thoracic aortic aneurysm, as described previously. Therefore, all of the aspects and characteristics relating to the method are applied to the corresponding system.

The first means for obtaining data linked to the individual's clinical and/or demographic characteristics can comprise typical clinical instruments, such as a blood pressure gauge, scales for measuring an individual's weight, diabetes readers, etc...

The second means for obtaining data linked to the biochemical characteristics of an individual's biological sample can comprise typical laboratory instruments suitable, for example, for analysing blood samples and identifying specific biomarkers.

The third means for obtaining data linked to the morphological and functional characteristics of the aorta can comprise instruments for CT, MR scans or ultrasound.

The fourth means can comprise computers running special simulation and analysis programs.

The system according to the invention represents a technological platform for the doctor, who is able to assess the weight of each single variable gathered during the clinical control phase of an individual, by means of artificial intelligence based on the concept of "machine learning", in order to quantify the risk of complications of the aneurysm. In particular, the system includes multidisciplinary and heterogeneous variables obtained, for example from the individual's computational modelling, from epigenetics, strain analysis, as well as family and personal history, lifestyle and risk factors predisposing to an aneurysm. The risk index is updated in real time when the individual's data is entered in the system and such parameter constitutes essential information for the doctor to determine the patient's therapy.

Such index is displayed on a graphic interface, in which a coloured scale represents the weight of each variable on the risk of complications of the aneurysm. The graphic interface of the CDSS instrument is developed using C# language, wherein libraries are adopted to access the archives of the data entered. A grouping system is adopted, which uses predefined rules to create the population of healthy and pathological individuals. The patient's variables are put in libraries by systemic sampling, wherein control protocols are used to stratify an individual's data. Such data sampling and grouping procedure constitutes the CDSS training phase. Once the CDSS training phase is complete, it is possible to enter a new case in the system and refine the predictive capacity of the system. The risk index is provided globally for the case in question, but also for every single variable so as to display the weight of each one.

These and other aspects of the present invention will become clearer in the light of the following description of some preferred embodiments described below.
- Fig. 1: shows a flow diagram of the method according to the present invention;
- Fig. 2: shows a schematic diagram in a block system of the system according to the present invention;
- Fig. 3: shows a diagram of a parametric model of the ATAA;
- Fig. 4: shows the steps for a numerical simulation of the ATAA;
- Fig. 5A-B: show the blood flows of an individual with an ATAA and TAV valve (a) of an individual with an ATAA and BAV valve (b);
- Fig. 6A-B: show the shear stress distributions of an individual with an ATAA and TAV valve (a) of an individual with an ATAA and BAV valve (b);
- Fig. 7A-D: show the values of shear stress (a) intramural stress (b) the helicoidal flow index (c) and the pressure index for an individual with an ATAA and TAV valve and an individual with an ATAA and BAV valve;
- Fig. 8A-B: show the correlation between shear stress (a) and intramural stress (b) with the aortic diameter of the vessel;
- Fig. 9: shows a mapping of the distribution of strain in individuals with ATAA and BAV valve and individuals with ATAA and TAV valve;
- Fig. 10: shows a diagram of the hierarchic CDSS structure according to the present invention.

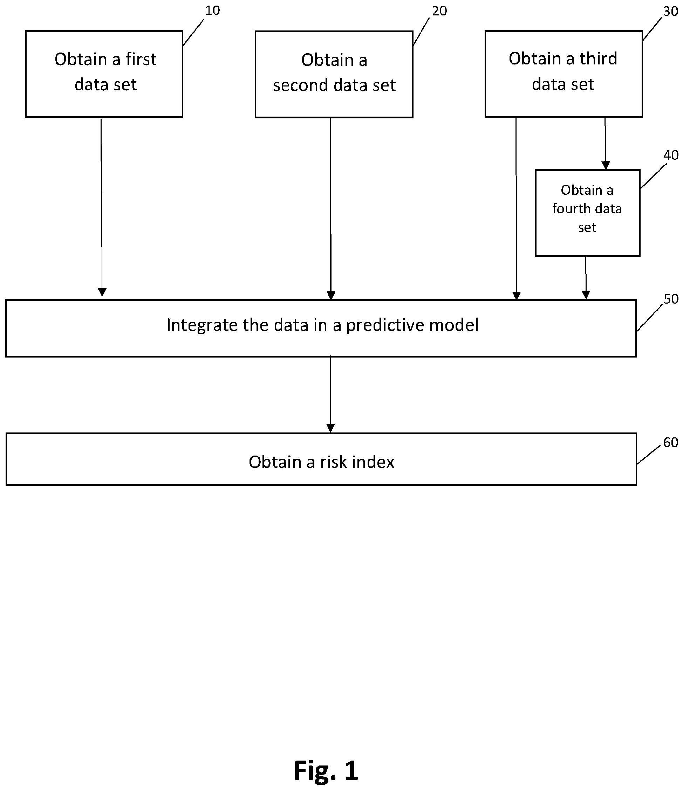

Figure 1 shows in a flow diagram the steps, which characterize the method according to the present invention. Assuming that an individual has been diagnosed with an ascending thoracic aortic aneurysm, the process starts by obtaining 10 a first data set 12 relating to the individual's clinical and/or demographic characteristics, obtaining 20 a second data set 22 relating to the biochemical characteristics of a biological sample taken from the individual and obtaining 30 a third data set 32 relating to the morphological and functional characteristics of the individual's aorta. The first data set 12 can be obtained immediately when the individual is admitted to hospital or to the clinic. With reference to the second data set 22, this can regard the creation of an epigenetic profile by special biomarkers (for example miRNA, MMP, TIMP) following a blood test carried out on the individual. To obtain the third data set 32 it is possible to use a CT, MR scan or an ultrasound of the individual's aorta. From the third data set 32, it is possible to obtain 40 a fourth data set 42. It is possible to estimate the hemodynamics and structural behaviour of the aneurysm by means of a computational simulation.

Then, the method comprises the step of integrating 50 the different variables relating to the first, the second, the third and the fourth data set 12, 22, 32, 42 in a predictive model. In particular, variables are integrated, which include, amongst others, the individual's demographic data, a personalized computational modelling of the hemodynamics and mechanics of the aneurysm, as well as biomarkers (for example epigenetic biomarkers) obtained from the circulating blood.

In this way, it is possible to obtain 60 a risk index i based on a physiological behaviour of the vessel and not only on a purely epidemiological criterion. It is worth noting that the predictive model is opportunely calibrated on a previously observed population. Furthermore, since the method is based on an automatic learning process, it is possible to evaluate the weight of each single variable gathered in the clinical control phase in order to quantify the risk of complications of the aneurysm.

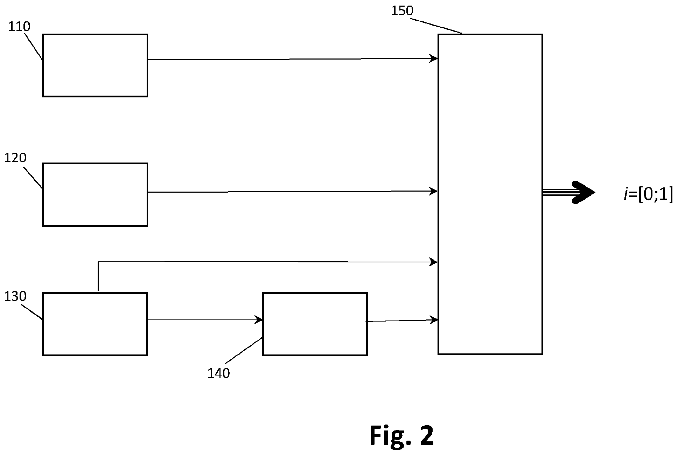

Figure 2 shows a schematic diagram of the system 100 according to the present invention. The system 100 essentially comprises means 110 for obtaining a first data set 12, means 120 for obtaining a second data set 22, means 130 for obtaining a third data set 32 and means 140 for obtaining a fourth data set 42. The data obtained from these means is entered in a computer 150 as input data, which processes it and issues a risk index i which can take a value from 0 to 1, wherein 0 represents no risk (0%) and 1 maximum risk (100%).

### Materials and methods

### Epigenetic profile

By analysing a sample of about 5 ml of blood taken by venipuncture, it is possible to evaluate the expression of the metalloproteinases (for example, MMP-1, -2, -3, -7, -8, -9), and the TIMP inhibitors thereof (for example, TIMP-1, -2, -3, -4), and the small endogenous molecules of non-coding RNA, in other words, miRNA. Examples of these molecules are miR-21 (access number: MI0000077) linked to endothelial damage, miR-143 (access number: MI0000459) and miR-145 (access number: MI0000461) linked to damage of the smooth-muscle tissue, miR-133a1 (access number: MI0000450) linked to cellular apoptosis, miR-155 (access number: MI0000681), and miR-16 (access number: MI0000070) linked to the inflammatory process of the aortic aneurysm, and finally miR-29b (access number: MI0000105) linked to tissue fibrosis.

The term "biological sample" refers in general to a sample of blood taken by venipuncture, but it can also include any other sample, now recognized or subsequently identified, which contains miRNA, MMP and TIMP such as (but not limited to) plasma, tissue or saliva or a combination thereof.

The identification of the expression of miRNA, MMP and TIMP according to the method described in the present invention is carried out by confirming the presence or absence of one or more miRNA in a biological sample. The miRNA, MMP and TIMP expression level of a patient with an ATAA can be determined using any method/technique recognized to-date as Next Generation Sequencing (for a broad spectrum analysis), polymerase chain reaction (PCR), PCR real-time or using a combination thereof. The difference in the expression of a miRNA, MMP and TIMP molecule between the biological sample of the individual with an ATAA and the biological sample of a healthy individual is indicative of the state of progress of the aneurysm.

A prospective study was carried out on a total of 32 patients with ATAAs and 16 controls (patients without an aneurysm) and subsequently on n.71 patients with an ATAA classified differently with BAV or TAV. All of the patients were followed at the hospital centre IRCCS Istituto Mediterraneo per i Trapianti e Terapie ad Alta Specializzazione (IRCCS ISMETT) (Mediterranean Institute for Highly Specialized Transplants and Therapies). Such study was approved by the local ethical committee with protocol IRB/04/14. After agreeing to take part in the study, blood samples were obtained from the patients by venipuncture and the expression of the serum levels of a kit of n.42 miRNA was assessed by PCR real-time with kit TaqMan® Array MicroRNA A + B Cards. The MMP-2, -3 and -9 and the TIMP-1, -2, -3 and -4 were also assessed.
ANOVA statistical analysis was carried out followed by a Holm-Sidak post-hoc test to compare the results of the patients with aneurysms with the controls. A significance was adopted for α=0.05.

Table 1 shows the average values (with the addition of standard deviation) for those miR (or miRNA) studied for the ATAAs, which showed a significant difference with the values of the control population. The miR were obtained by PCR real-time on aneurysms and controls. The units of measurement are 2^-ddCT, while U16 was chosen for reference.
In detail, of the n.42 miR studied, statistically significant differences were obtained for n.8 miR (table 1).

**Table 1: Comparison of the statistically significant miR expression levels between ATAAs and controls (healthy individuals)**

| | **Controls** (n=16) | **ATAA** (n=40) | **p** |
|---|---|---|---|
| ***miR-16 (MI0000070)*** | 1.13±1.08 | 6.16±3.86 | 0.003 |
| ***miR-9*** | 1.47±2.02 | 5.15±3.68 | 0.023 |
| ***(MI0000466)*** | | | |
| ***miR-101 (MI0028598)*** | 2.79±5.25 | 17.7±12.0 | 0.005 |
| ***miR-143 (MI0000459)*** | 0.79±0.59 | 21.8±22.61 | 0.025 |
| ***miR-19 (MI0028679)*** | 1.00±0.83 | 13.4±27.38 | 0.002 |
| ***miR-21 (MI0000077)*** | 2.15±2.86 | 22.6±21.68 | 0.023 |
| ***miR-29 (MI0000105)*** | 1.35±1.01 | 4.5±2.71 | 0.008 |
| ***miR-423-5p (MI0001445)*** | 1.64±1.49 | 6.00±5.1 | 0.042 |

Furthermore, a different expression was found for the following n.5 miR among patients with BAV versus TAV (table 2) on a new population of 71 patients with an ATAA.

**Table 2: Statistically significant miR expression levels of ATAAs with TAV compared to ATAAs with BAV**

| | **ATAA BAV** (n=24) | **ATAA TAV** (n=47) | **p** |
|---|---|---|---|
| ***miR-133 (MI0000450)*** | 1.48±1.508 | 3.12±2.45 | 0.007 |
| ***miR-155 (MI0000681)*** | 0.74±3.83 | 2.73±2.91 | 0.032 |
| ***miR-320a. (MI0000542)*** | -1.08±2.32 | 0.32±2.11 | 0.033 |
| ***miR-34a (MI0001251)*** | -0.26±2.51 | 1.27±2.15 | 0.029 |
| ***miR-34a (MI0000268)*** | -0.12±2.98 | 2.20±1.69 | 0.002 |

Such miR distinguish the presence of an ATAA in an individual with a TAV from a BAV, with an increased risk of rupture/dissection of the vessel. The miR-34a (MI0000268) has been correlated significantly (R=-0.843, p=0.001) with the elastic capacity of the vessel (i.e., stiffness) assessed as the variation in diameter of the vessel in a heartbeat in relation to the variation in blood pressure between systole and diastole. Logistic regression has shown that miR-34a (MI0000268) predicts the presence of an ATAA when age and smoking are used as confusing variables (β=-0.131, p=0.017, 95% CI upper bound=0.022 and lower bound= 26.04).

Table 3 shows the differences for MMP and TIMP values, which showed a statistically significant difference. In particular, only MMP-9 and TIMP-1 are significant for the decision support. In this case, too, the average is reported with standard deviation and the units of measurement are pg/mL.

**Table 3: Comparison of the statistically significant absolute values of the metalloproteinases and tissue inhibitors between controls (healthy individuals) and ATAAs**

| | **Controls** (n=16) | **ATAA** (n=40) | **p** |
|---|---|---|---|
| ***MMP-9** (1x10²) **(AAD37404.1)*** | 0.63±0,500 | 18.54±12.5 | 0.005 |
| ***TIMP-1** (1x10²) **(CAG46779.1)*** | 33.28±14.76 | 52.08±13.55 | 0.001 |

The conclusion is that such identified molecules of miR, metalloproteinases and respective inhibitors are altered in patients with an aneurysm compared to healthy patients and that they thus have a prognostic significance in the state of progress of the disease.

C-reactive protein (access number: NP_000558; version: NP_000558.2) determined from serum or heparinized plasma. creatine kinase (access number: NP_001814; version: NP_001814.2), Nt-proBNP (access number: NP_002512; version: NP_002512.1), cardiac troponin I (access number: NP_000354; version: NP_000354.4) and interleukin 6 (access number: NP_000591; version: NP_000591.1) determined from serum or plasma. All of these proteins are evaluated using chemiluminescence technology.

Advanced glycation end product AGE (access number:P51606; version:P51606.2) and corresponding receptor RAGE (access number:ACF47656; version:ACF47656.1), transforming growth factor beta TGF-beta (access number: NP_000651; version: NP_000651.3), D-dimer (access number: 2Q9I_F; version: 2Q9I_F) determined by kit for enzyme linked (ELISA) immuno-absorbent assay.

### Computational modelling and numerical modelling

Using DICOM data (Digital Imaging and COmmunications in Medicine) of a CT or MR scan, a process of semi-automatic "reverse engineering" is carried out, based on operations of segmentation and thresholding to reconstruct the anatomy of the vessel including a) the aortic valve with the spatial position of the cusps of the valve, b) the ascending aorta, b) the aortic arch with the supra-aortic trunks, c) the aorta descending until iliac level. This process can be carried out using open-source software, such as, for example vascular tool ITK. For an accurate reconstruction of the morphology of the aortic valve, it is advisable to perform a CT angiography. However, in the absence of this, it is possible to use a parametric model to model the aortic valve, both for a TAV and a BAV, using echocardiographic measurements of the valvular orifice area, the size and spatial position of the cusps.

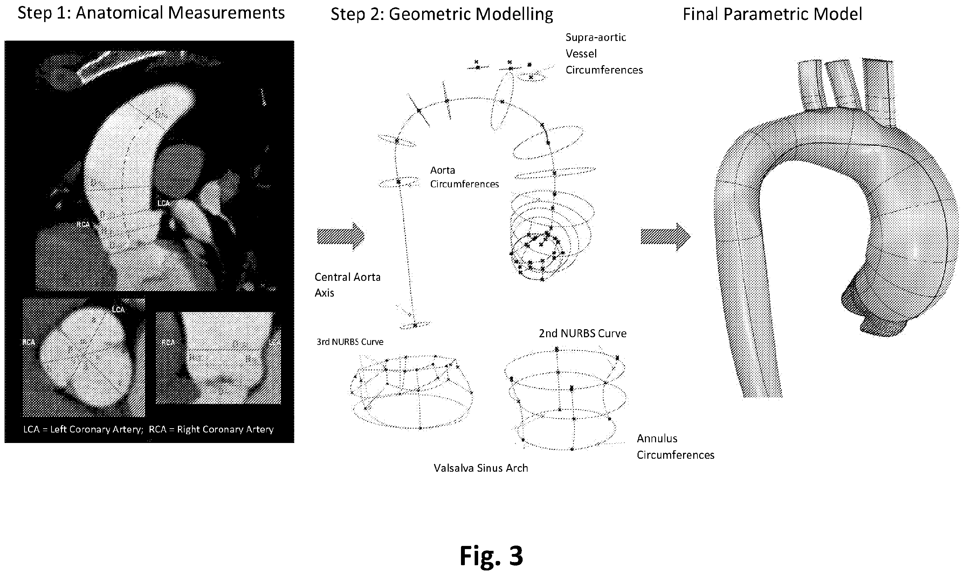

Figure 3 shows the steps for obtaining a parametric model of the ATAA. The first step comprises the anatomical measurements of the aorta. The second step comprises geometrical modelling and the third step represents the final output parametric model.

It is possible to produce a virtual parametric geometry of the aorta and the valve using CAD modelling techniques (computer-aided design) based on NURBS surfaces. In this case, it is first necessary to have the echocardiographic measurements of the aortic valve and the aorta in different anatomical regions along the longitudinal direction thereof, as shown in figure 3. These measurements are: a) diameter of the annulus (D_{An}); b) the diameter of the sinuses of Valsalva (D_{Sin}) ; c) the diameter of the tubular junction (D_{STJ}) ; d) n.8 diameters of the aorta equally spaced along the longitudinal direction (D_{A*oi*} con *i=1,8*); e) the distance between the annulus and the sinuses (Hₛᵢₙ); f) the distance between the annulus and the tubular junction (H_{STJ}); g) the intercommissural distance of each sinus of Valsalva (a, b, c) and h) the corresponding corners on the valve plane (α, β, γ, δ, ε).
In short, the aortic annulus and the sino-tubular junction can be described by a circular shape. The distance between the annulus and the sino-tubular junction is used to position these two circumferences in the space. Whereas, the sinuses of Valsalva can be described by semi-circumferences obtained by interpolating the end points of the intercommissural distance of the aortic valve.
To model a TAV, it is necessary to use three NURBS surfaces of the third order interpolation to model the leaflets or cusps of the valve. It is possible to adjust the convexity of such leaflets of the valve by check points of the NURBS surface based on the images shown by the transesophageal echocardiography. To model the BAV, two NURBS surfaces of the third order interpolation are needed. Three check points in a system of cylindrical coordinates are created mid-height of each cusp, to control the curvature of the valve cusps. These NURBS surfaces are constrained to the surface of the aortic sinus by morphological operations.

To generate the geometry of the ascending aorta, the spatial distribution of the diameters of the aorta is used measured along the central axis of the vessel based on the multi-planar views (in other words, sagittal, coronal and axial) of the echocardiographic image. The aorta is assumed to have a circular shape in the transversal plane along the central axis of the vessel. A "loft profusion" is used to generate a surface, which interpoles the n.8 diameters of the aorta along the central axis of the vessel previously measured by the transesophageal ultrasound. Whereas, the surfaces of the supra-aortic vessels are modelled by means of "loft protrusions" of the circle, which identifies the diameter of each supra-aortic vessel. This is shown in figure 3.

The numerical simulation according to the present invention adopts the finite elements method (FEM) as numerical technology for the solution of the differential equations for the partial derivatives, which govern the movement of the fluids and aortic mechanics. The numerical solution is carried out using commercial FEM packages.
The virtual geometry of the anatomy of the aorta is rendered discrete in small elements of finite volume (about 1 million tetrahedral elements for fluid dominion and about 30 thousand quadrilateral-shaped shell elements for structural dominion). The method according to the present invention is based on a bi-directional fluid-structure analysis using MpCCI software (Fraunhofer SCAI, Germany) for coupling the structural component (ABAQUS, SIMULIA Inc., Providence, RI), with the math solver of the fluid movement, (FLUENT, ANSYS Inc., Canonsburg, PA). The FLUENT and ABAQUS codes share a common border area where the data exchange takes place. The MpCCI algorithm allows data to be exchanged on meshes of non-corresponding elements, by interpolation on the nodes of data obtained from each code.

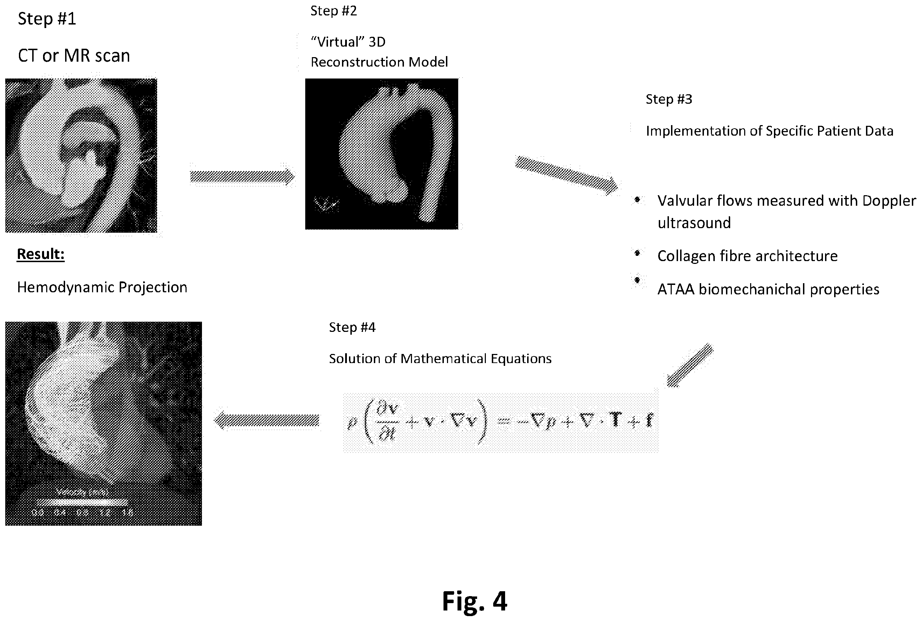

In ABAQUS, the biomechanical behaviour of the aorta is modelled as a hyper-plastic and homogeneous material and uses material parameters determined by mechanical tests on samples of patients with BAV or TAV, who have undergone surgical repair of the ATAA. Such biomechanical behaviour model of the aorta considers the dispersion of collagen fibres, which is typical of an aneurysm. The thickness of the vessel (about 1.8 mm for the BAVs and 2.0 mm for TAVs) are assumed as constant. A dynamic/implicit formulation is used to solve the math of the equations, which define the mechanical behaviour of the vessel because of the considerable deformation of the vessel itself. In order for the aorta to deform physiologically, the distal ends of the supra-aortic trunks, the aortic valve and the descending aorta are constrained in all directions.
In a FLUENT environment, a transitory analysis is carried out for the simulation of the fluid dynamics of the movement of the blood. The blood is assumed to be laminar, incompressible and Newtonian with a density of 1060 kg/m3 and a viscosity of 0.00371 Pa x s. PISO is used as a pressure-speed coupling algorithm to improve convergence in the immediate vicinity of the distorted elements and the PRESTO scheme as pressure interpolation method. The convergence of the solution is obtained when the remainder of the continuity equation reaches 10-5. The measurement of the transaortic flow is used as the inlet speed of the flow in the aortic valve, measured with a standard echocardiographic exam, which is carried out on the patient as part of their treatment. Whereas, a model with concentrated parameters of the systemic circulation is used for flows leaving the supra-aortic trunks and the abdominal aorta using the blood pressure measurements with a mercury sphygmomanometer. Figure 4 shows the steps needed for the computational modelling. The first step consists of the acquisition or scanning of CT or MR images. The second step consists of the reconstruction of the virtual anatomy of the aorta in 3D. The third step comprises the inclusion and implementation of data relating to the specific individual like the valvular flow by eco-Doppler, the collagen fibre architecture and the biomechanical properties of the aortic wall. The fourth step includes the application of math equations (Navier-Stokes differential equations). The final step or fifth step consists of the hemodynamics projection, which constitutes the modelling output.

The post-processing of the results of the computational simulation consists of a) displaying the hemodynamic and structural parameters superimposing them on the patient's virtual anatomy and b) extrapolating these parameters in different anatomical positions of the vessel. Examples of such parameters are blood pressure, shear stress and intramural stress. A coloured map of the parameter of interest is used wherein the colour red indicates, for example a high value (significant risk) while blue indicates, for example a low value (negligible risk).
Furthermore, the average values of such parameters are extrapolated in different anatomical areas, including the sinus of Valsalva, the sino-tubular junction, the proximal part of the ascending aorta. In particular, the following hemodynamic and structural variables are assessed for every simulation a) shear stress; b) the pressure index described by the 95% highest value of the pressures normalized by the peak thereof; c) the helicoidal flow index as an indicator of three-dimensionality of the flow; d) intramural stress (in terms of Von Mises stress) for the layers of the internal and external tunica of the aorta.

A retrospective assessment was carried out on a total of 78 patients with ATAA BAV valve (n = 42) and ATAA with TAV valve (n = 36) followed at the IRCCS Mediterranean Institute for Highly Specialized Transplants and Therapies (ISMETT IRCCS). The study was approved by the ethical committee with protocol IRB/04/14. The following inclusion criteria was used: individuals with ATAAs aged >18 years old. The following exclusion criteria was used: severe high blood pressure; connective tissue disorders; clinical history of surgical operations; aortic stenosis (AS) or aortic insufficiency (AR) more than mild. The classification schemes of BAV aortic valve and the aorta morphology suggested by Schaefer et al were used. The bicuspid aortic valve: an integrated phenotypic classification of leaflet morphology and aortic root shape. Heart 2008;94:1634-8. Table 4 summarizes the demographic data and the results of the descriptive statistics.

**Table 4: Demographic characteristics of patients with an ATAA**

| | **BAV ATAA** (n=42) | **TAV ATAA** (n=36) | **p-value** |
|---|---|---|---|
| ***age,** years* | 58±13 | 65±9 | 0,061 |
| ***Sex** (%)* | 76 | 23 | 0.004 |
| ***AR** (%)* | 76 | 44 | 0.139 |
| ***AS** (%)* | 10 | 23 | 0.348 |

| ***Aortic Diameters*** (mm) | | | |
|---|---|---|---|
| ***sinuses*** | 37.1±4.7 | 38.8±2.6 | 0.676 |
| ***Sino-tubular Junction*** | 35.4±6.2 | 38.9±6.6 | 0.132 |
| ***Aorta*** | 42.7±5.3 | 45.4±10.0 | 0.451 |

| ***Morphology of the aorta** (n)* | | | |
|---|---|---|---|
| ***Type N*** | 2 | 3 | |
| ***Type A*** | 15 | 7 | |
| ***Type E*** | 4 | 3 | |

| ***BAV Morphology** (n)* | | | |
|---|---|---|---|
| ***Type 1*** | 14 | / | |
| ***Type 2*** | 7 | / | |
| ***Orifice Area** (mm2)* | 346.2±88.6 | 447.8±75.8 | 0.003 |
| ***Transaortic Jet*** (m/s) | 2.0±0.8 | 1.7±0.6 | 0.124 |

Computational modelling was carried out as described in this invention to assess the hemodynamics and structural mechanics of ATAAs.

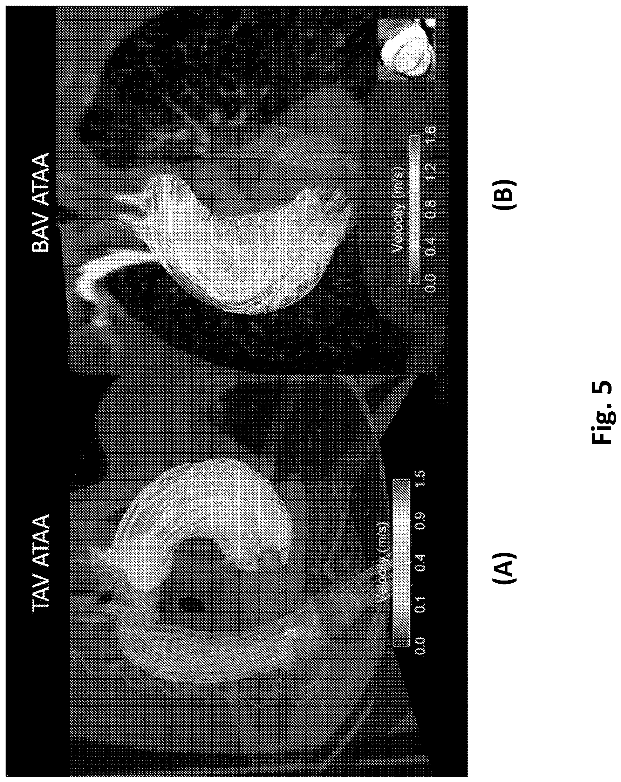

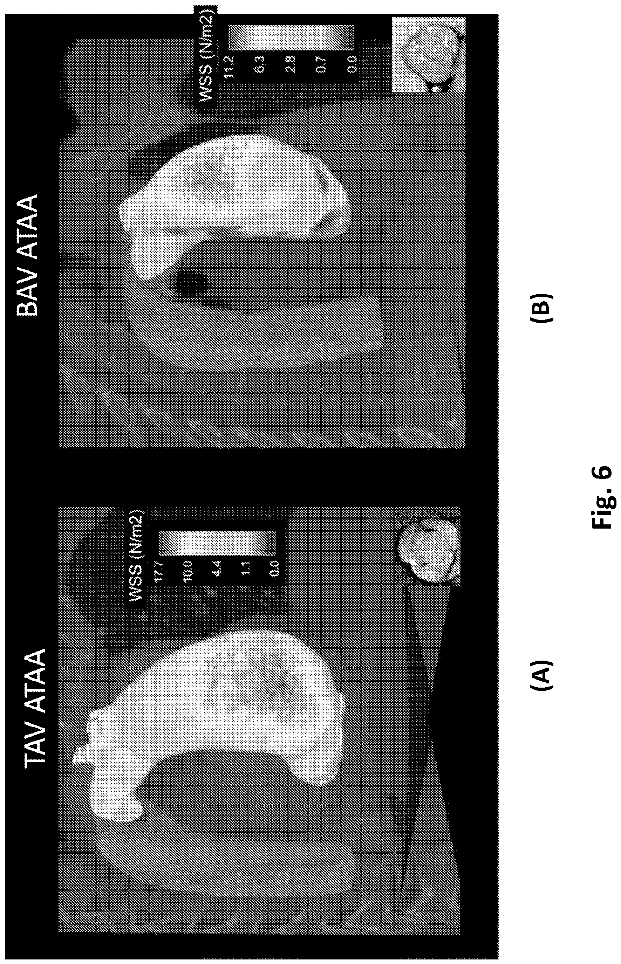

Figure 5 shows the hemodynamics of two patients with an aneurysm and different aortic valve morphology, while Figure 6 shows the distribution of shear stress induced by the blood flow.

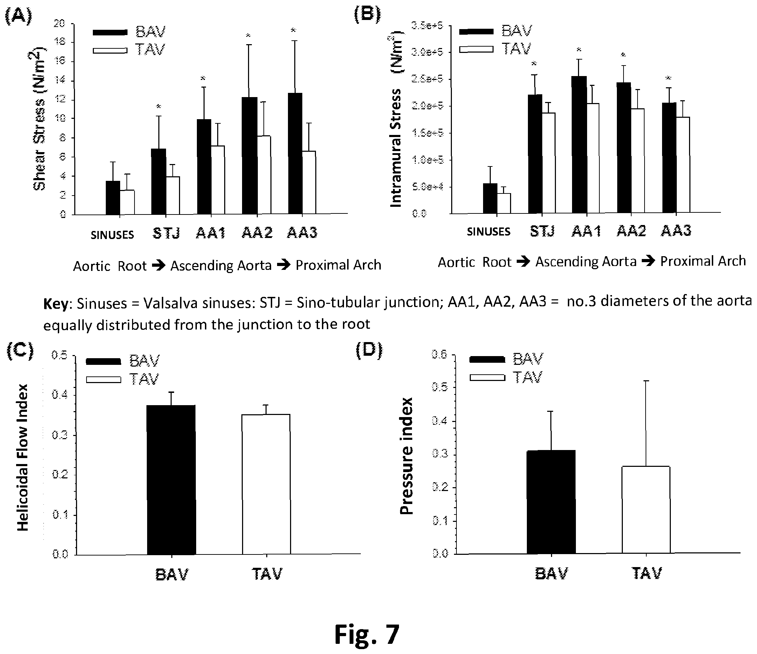

Figure 7 shows the bar diagrams of the average values (plus standard deviation) of the computational variables for the two populations in question. In particular, figure 7A shows the shear stress values and figure 7B the intramural stress and strain values in different positions of the aorta. Figures 7C and 7D show the helicoidal and pressure flow index values respectively. The significant statistical difference is p<0.05.

The results highlight shear stress (WSS) of the ATAAs with a higher BAV than the one observed in patients with TAV in the sino-tubular junction (6.8 ± 3.3 N/m2 for BAV and 3.9 ± 1.3 N/m2 for TAV, p=0.006) and in the ascending aorta (9.8 ± 3.3 N/m2 for BAV and 7.1 ± 2.3 N/m2 for TAV, p = 0.040, figure 7A). A statistically significant difference was observed in the BAVs compared to the TAVs for intramural stress along the ascending aorta (for example, 2.54x105 ± 0.32x105 N/m2 for BAV and 2.04x105 ± 0.34x105 N/m2 for TAV, p<0.001, figure 7B). The hemodynamics appears more disorganized for patients with BAV than in patients with TAV, although not statistically significant.

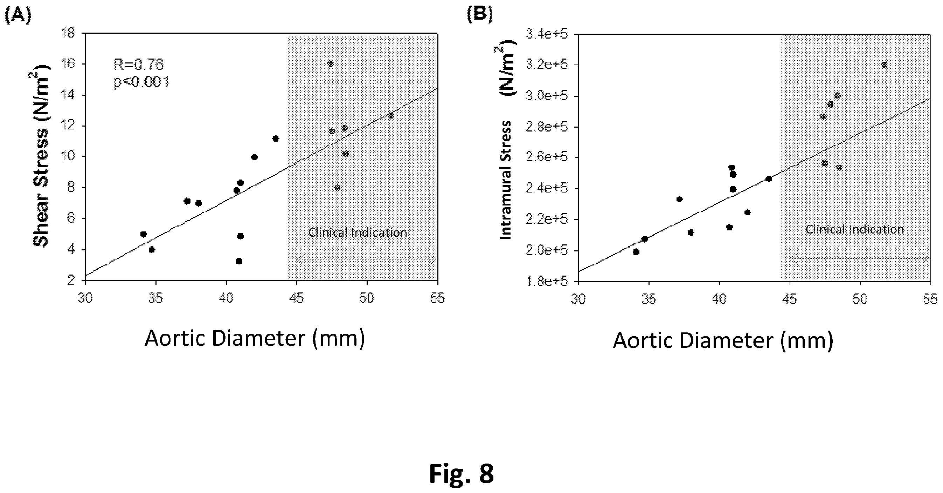

Figure 8 shows the correlation between shear stress (figure 8A) and intramural stress (figure 8B) with the aortic diameter of the vessel. The results show a statistically significant Pearson correlation between shear stress and the diameter of the ascending aorta (R = 0.76, p = 0.002. Similarly, it is possible to note a significant correlation between intramural stress and the diameter of the ascending aorta (R = 0.89, p = 0.003). It is interesting to note that, patients with an ATAA and BAV valve who undergo surgery have higher intramural stress than patients who have not undergone an operation, suggesting that such parameter takes on prognostic significance to distinguish a malign aneurysm from a benign one, as shown by the indications in figures 8.

In conclusion, patients with an ATAA and BAV valve show significantly higher shear stress and intramural tension values than patients with an ATAA and TAV valve when these two groups of patients are compared at the same age and size of the aneurysm. This study was carried out by lowering the effect of the variables, which could confuse the progress of the aneurysm between the two populations and this shows that the differences in the parameters of shear stress and intramural stress are intrinsic for patients with a BAV valve compared to those with a morphologically normal valve. This highlights the importance of using the computational parameters to identify highly stressed areas of the aortic wall, which are consequently at a higher risk of developing complications and thus in need of more attention by the doctor.

### Time tracking algorithm

A knowledge of the kinematics of the aortic wall is of considerable importance for assessing the physiopathology of the ATAA. Although a CT angiography is not the standard instrument for aneurysm imaging, this technology is advisable because it allows the size of the aneurysm to be measured both in diastole (in other words, when blood pressure is low and intramural stress is low) and in systole (in other words, when blood pressure is high and intramural stress is high). A CT angiography allows the quantification of parameters, such as deformation (also known as "strain") - a parameter correlated to cardiac dysfunction and the progression of the disease in some heart pathologies.

It is possible to extrapolate the morphology of the aorta in different instants of time of the heartbeat from a CT angiography and consequently apply an algorithm for the temporal monitoring of the wall based on analysis techniques of the movement and temporal recognition. This allows the field of movement of the aorta to be estimated for every heartbeat image. The deformation is thus expressed in relation to the initial configuration of the vessel, which is obtained from the image with the highest vessel contraction. It is also possible to calculate the deformation speed (in other words, the "strain rate") in relation to the systole time. These parameters can be mapped with a coloured scale on the virtual geometry of the aorta as described previously.

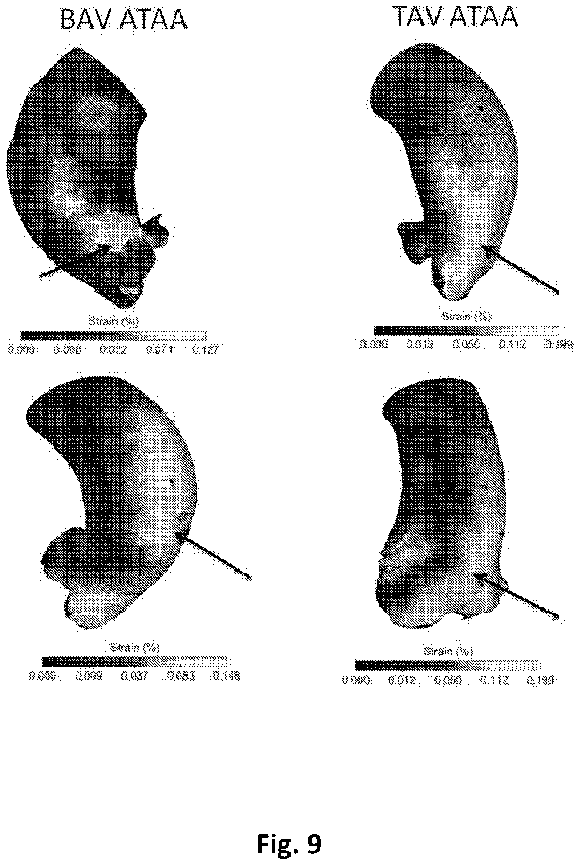

A retrospective assessment was carried out on a sample of 14 patients with ATAAs both with BAV valve and TAV valve followed at the IRCCS Mediterranean Institute for Highly Specialized Transplants and Therapies (ISMETT). The study was approved by the ethical committee with protocol IRB/04/14.
Patients without aortic dilation were also enlisted as a negative control and to compare with the data of patients with an ATAA. The patients underwent a CT angiography, according to the radiologists' clinical indications, and not for the specific purpose of this invention. The time tracking algorithm was used to obtain the strain and strain rate of the patients enlisted. Table 5 shows the average values of these two parameters, while Figure 9, the distribution of the deformation of the ATAA.

**Table 5: Comparison of the strain parameter between controls (healthy individuals) and ATAA with BAV or TAV as an indicator of the elasticity of the pathological tissue**

| | **Controls** (n=5) | **BAV ATAA** (n=6) | **TAV ATAA** (n=8) |
|---|---|---|---|
| ***Strain,** %* | 0.08±0.03 | 0.16±0.04 | 0.13±0.06 |
| ***Strain Rate,** 1*/*s⁻¹* | 0.44±0.09 | 0.53±0.14 | 0.49±0.19 |

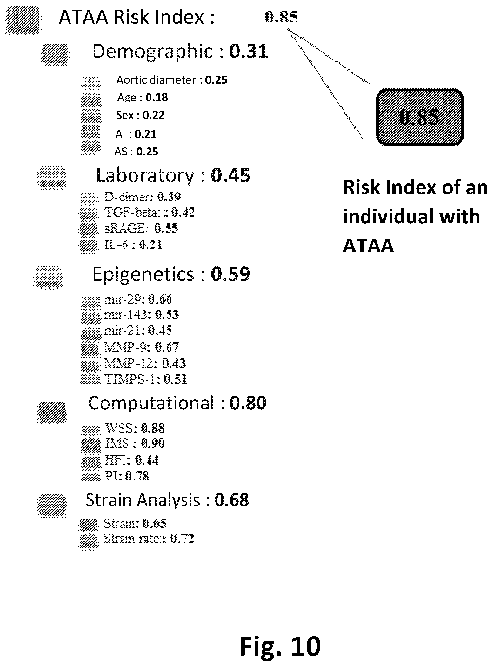

Figure 10 shows the hierarchical structure of the method or system according to the present invention. All of the data is represented in a hierarchy tree view, using colours and shapes to quickly distinguish the patient's condition and the importance of all of the variables entered. These are the variables relating to the first data set (12), the second data set (22), the third data set (32) and the fourth data set (42). In particular, the risk index of the aneurysm is highlighted in figure 10 for a clinical scenario of a 55-year-old patient with a bicuspid valve and aortic ectasia of 3.7 cm, in other words, an aortic dilation, which is not clinically significant and consequently does not require immediate surgery. Such aortic dilation was identified after a first echocardiographic exam, showing good functionality of the valve, which was subsequently confirmed by a CT angiography. Based on the individual's clinical history, the doctor recommends a radiological examination in six months to assess the progress of the aortic dilation. Whereas, the doctor adopts the CDSS presented in this invention to assess the risk for the patient in question. After collecting all of the data, figure 10 shows the result of the CDSS, which informs the doctor that there is a potentially high risk of complications related to aortic dilation (being 0.86 out of a maximum of 1). It can be noted from figure 10 that the weight of the computational data compared to, for example, the demographic data is high, probably due to considerable hemodynamic alterations induced by the conformation of the bicuspid valve. Therefore, the doctor, who, thanks to the CDSS, has much more information compared to that of the current criterion of the maximum diameter of the aorta, can review the therapy and decide to intervene immediately to avoid the risk of complications caused by the dilation.

In other words, the risk index obtained using the method according to the present invention uses variables to model the state of progress of the disease. The method transforms the multidisciplinary variables in a common space and aggregates them to obtain the end result. Therefore, an iterative calculation is used to improve the predictive capacity of the model itself and thus the output is represented by the risk index, which is represented in a hierarchy tree view to display the weight, which each variable has on the state of progress of the disease.

The highly innovative aspect of the present invention lies in the combination of data from biomarkers, such as, for example epigenetic data and computational calculations for a personalized stratification for the patient in question, proposing a method and system for a more rigorous decision-making, to distinguish a "benign" aneurysm from a "malign" one, reliably and accurately. Any type of radiological imaging can be used (for example, CAT, magnetic resonance or echocardiography), epigenetic biomarkers, analysis of deformations of the vessel in order to consider information heterogeneous and of any type or scale. Thus, a new paradigm of predictive medicine is proposed, wherein the doctor can benefit from the advantages of a decision instrument, which reconstructs a virtual model of the anatomy of the individual's aneurysm, based on radiological imaging and thus uses numerical simulation technology and biomarkers to quantify the physiological behaviour of the vessel and the risk associated with aneurysm.

A study, for example on the epigenetic profile of a sample of patients with an ATAA, showed that miRNA molecules obtained from peripheral blood are able to distinguish patients with an aneurysm from healthy patients. Thus, miRNA expression levels have a potential for clinically stratifying patients with an aneurysm. Similarly, degradation of the extracellular matrix is associated with the concentration of MMP, while TIMP inhibitors are important regulators of MMP activity. Thus, the assessment of the concentration of MMP and TIMPS from circulating blood represents a simple method for identifying and monitoring individuals with an ATAA and BAV valve. However, the heterogeneity of the disease does not allow only the measurements of the concentration of MMP and TIMP to be used for the clinical stratification of the aneurysm.

The computational study in a certain number of patients with ATAAs and different valve morphology showed that the method and system according to the present invention is able to provide important hemodynamic and structural parameters to identify areas of the aortic wall with a higher risk of complications. Computational modelling can allow the development of a new technology for a personalized approach in diagnosing and managing the diseases compared to the traditional guidelines based on epidemiological information. The great innovation of the computational method used is that of integrating structural characteristics of the behaviour of the vessel, specific of an aneurysm and the valve thereof, in a fluid-structure model, which are unique compared to the theoretical assumptions of the other computational analyses. To distinguish the differences between BAV and TAV, the computational model considers the inherent defect of the collagen fibres of the wall of the aneurysmatic aorta and the difference in the mechanical response of the vessel itself. Finally, an analysis of the deformations using a time tracking algorithm represents an *in-vivo* assessment of the mechanical behaviour of the wall of the vessel and a rapid way of quantifying the structural parameters linked to the presence of an aneurysm.

An important aspect of the method and system according to the present invention is that of defining artificial intelligence (in other words, an automatic learning system), which is able to provide a parameter essential for optimizing the therapeutic approach of a patient with an ATAA by integrating multidisciplinary and heterogeneous data. In the context of pathologies, for example cancer or Alzheimer's disease, studies have shown that such a support instrument can transform all of the information on a patient in a practical manner, generating knowledge, which can be applied in a clinical setting.

Numerous further modifications and variations can be made to the method and system described above by a person skilled in the art with the aim of satisfying further, contingent needs, all comprised within the protective scope of the present invention, as defined by the appended claims.

## Claims

1. A method for calculating a risk index of aortic rupture or dissection of an individual with ascending thoracic aortic aneurysm, ATAA, the method comprising the steps of:
obtaining (10) a first data set (12) linked to the clinical and/or demographic characteristics of the individual;
obtaining (20) a second data set (22) linked to the biochemical characteristics of a biological sample of the individual;
obtaining (30) a third data set (32) linked to the morphological and functional characteristics of the aorta and processing said third data set (32) to obtain (40) a fourth data set (42) by computational modelling; and
integrating the first data set (12), the second data set (22), the third data set (32) and the fourth data set (42) in a predictive model to obtain the risk index (i) of aortic rupture or dissection;
**characterized in that**
the second data set (22) comprises expression values of at least one non-coding RNA biomarker chosen from the group comprising: miR-16, miR-9 miR-101, miR-143, miR-19, miR-21, miR-29, and miR-423-5p.

2. The method according to claim 1, wherein the biomarker is chosen from the group comprising: a metalloproteinase of the extracellular matrix, MMP, and a tissue inhibitor, TIMP.

3. The method according to claim 1 or 2, wherein the second data set (22) further comprises expression values of at least one biomarker of non-coding RNA chosen from the group comprising: miR-133a, miR-155, miR-320a, miR-34a (MI0001251), miR-34a (MI0000268) .

4. The method according to claim 2 or 3, wherein the metalloproteinase of the extracellular matrix is MMP-9 and the tissue inhibitor is TIMP-1.

5. The method according to one of the preceding claims, wherein the biomarker is chosen from the group comprising: C-reactive protein, creatine kinase, Nt-proBNP, troponin, advanced glycation end product, AGE, and corresponding receptor, RAGE, transforming growth factor-beta, D-dimer and interleukin 6, IL-6.

6. The method according to one of the preceding claims, wherein the third data set (32) comprises morphological data following a virtual reconstruction of the individual's aortic anatomy by a diagnostic imaging method.

7. The method according to one of the preceding claims, wherein the fourth data set (42) comprises hemodynamic and structural parameters of the aorta estimated by a numerical simulation and wherein said hemodynamic and structural parameters are integrated in a bi-directional fluid-structure model.

8. The method according to claims 6 and 7, further comprising a processing of the numerical simulation results to display the hemodynamic and structural parameters superimposing them on the virtual reconstruction of the aortic anatomy and extrapolating said parameters in different anatomic positions of the aorta.

9. The method according to claim 7 or 8, wherein the hemodynamic and structural parameters comprise at least the blood pressure, shear stress, intramural stress and helicoidal flow index.

10. The method according to one of the preceding claims, wherein the fourth data set (42) further comprises information relating to a deformation of the aorta and a time variation of said deformation obtained by applying a time tracking algorithm.

11. The method according to one of the preceding claims, further comprising an assessment of the weight of each datum belonging to the first, second, third or fourth data set (12, 22, 32, 42) on the risk index (i).

12. A system (100) for calculating a risk index of aortic rupture or dissection of an individual with ascending thoracic aortic aneurysm, ATAA, the system comprising: first means (110) to obtain a first data set (12) linked to the clinical and/or demographic characteristics of the individual; second means (120) to obtain a second data set (22) linked to the biochemical characteristics of a biological sample of the individual; third means (130) to obtain a third data set (32) linked to the morphological and functional characteristics of the aorta; fourth means (140) to obtain a fourth data set (42) obtained from a processing of the third data set (32) by means of computational modelling; and a computer (150) having a data interface for receiving the first data set (12) the second data set (22), the third data set (32) and the fourth data set (42) as input data and a processor for processing said data (12, 22, 32, 42) and issuing the risk index (i) of aortic rupture or dissection as output data, integrating the first, the second, the third and the fourth data set (12, 22, 32, 42) in a predictive model, wherein the second data set (22) comprises expression values of at least one biomarker of non-coding RNA chosen from the group comprising: miR-16, miR-9 miR-101, miR-143, miR-19, miR-21, miR-29, and miR-423-5p.

13. The system according to claim 12, wherein the second data set (22) further comprises expression values of at least one biomarker of Non-coding RNA chosen from the group comprising: miR-133a, miR-155, miR-320a, miR-34a (MI0001251), miR-34a (MI0000268) .

## Patentansprüche

1. Verfahren zum Berechnen eines Risikoindexes für eine Aortenruptur oder -dissektion bei einem Individuum mit thorakalem Aneurysma der Aorta ascendens (ATAA - ascending thoracic aortic aneurysm), wobei das Verfahren folgende Schritte umfasst:
Erhalten (10) eines ersten Datensatzes (12) in direktem Zusammenhang mit den klinischen und/oder demografischen Eigenschaften des Individuums;
Erhalten (20) eines zweiten Datensatzes (22) in direktem Zusammenhang mit den biochemischen Eigenschaften einer biologischen Probe des Individuums;
Erhalten (30) eines dritten Datensatzes (32) in direktem Zusammenhang mit den morphologischen und funktionellen Eigenschaften der Aorta und Verarbeiten des dritten Datensatzes (32), um einen vierten Datensatz (42) durch Modellrechnungen zu erhalten (40); und
Integrieren des ersten Datensatzes (12), des zweiten Datensatzes (22), des dritten Datensatzes (32) und des vierten Datensatz (42) in einem Vorhersagemodell, um den Risikoindex (i) der Aortenruptur oder -dissektion zu erhalten;
**dadurch gekennzeichnet, dass**
der zweite Datensatz (22) Expressionswerte von mindestens einem nichtkodierenden RNA-Biomarker umfasst, ausgewählt aus der Gruppe, die folgende umfasst: miR-16, miR-9, miR-101, miR-143, miR-19, miR-21, miR-29 und miR-423-5p.

2. Verfahren nach Anspruch 1, wobei der Biomarker aus der Gruppe, die folgende umfasst ausgewählt ist: eine Metallproteinase der extrazellulären Matrix (MMP) und ein Gewebeinhibitor (TIMP).

3. Verfahren nach Anspruch 1 oder 2, wobei der zweite Datensatz (22) ferner Expressionswerte von mindestens einem Biomarker der nichtkodierenden RNA umfasst, ausgewählt aus der Gruppe, die folgende umfasst: miR-133a, miR-155, miR-320a, miR-34a (MI0001251), miR-34a (MI0000268) .

4. Verfahren nach Anspruch 2 oder 3, wobei die Metallproteinase der extrazellulären Matrix MMP-9 ist und der Gewebeinhibitor TIMP-1 ist.

5. Verfahren nach einem der vorhergehenden Ansprüche, wobei der Biomarker aus der Gruppe, die folgende umfasst ausgewählt ist: C-reaktives Protein, Kreatinkinase, Nt-proBNP, Troponin, Advanced Glycation End Products (AGE) und entsprechender Rezeptor (RAGE), Transforming Growth Factor beta, D-Dimer und Interleukin 6 (IL-6).

6. Verfahren nach einem der vorhergehenden Ansprüche, wobei der dritte Datensatz (32) morphologische Daten nach einer virtuellen Rekonstruktion der Aortenanatomie des Individuums durch ein diagnostisches Bildgebungsverfahren umfasst.

7. Verfahren nach einem der vorhergehenden Ansprüche, wobei der vierte Datensatz (42) hämodynamische und strukturelle Parameter der Aorta umfasst, die von einer numerischen Simulation geschätzt werden, und wobei die hämodynamischen und strukturellen Parameter in einem bidirektionalen Fluid-Struktur-Modell integriert sind.

8. Verfahren nach Anspruch 6 und 7, ferner umfassend eine Verarbeitung der Ergebnisse der numerischen Simulation, um die hämodynamischen und strukturellen Parameter darzustellen und sie über die virtuelle Rekonstruktion der Aortenanatomie zu legen und die Parameter an verschiedenen anatomischen Stellen der Aorta zu extrapolieren.

9. Verfahren nach Anspruch 7 oder 8, wobei die hämodynamischen und strukturellen Parameter mindestens den Blutdruck, die Scherbeanspruchung, die intramurale Beanspruchung und den helikalen Fließindex umfassen.

10. Verfahren nach einem der vorhergehenden Ansprüche, wobei der vierte Datensatz (42) ferner Informationen bezüglich einer Verformung der Aorta und eines Zeitverlaufs der Verformung, er durch das Anwenden eines Zeiterfassungsalgorithmus erhalten wird, umfasst.

11. Verfahren nach einem der vorhergehenden Ansprüche, ferner umfassend eine Beurteilung des Gewichts jedes Bezugspunkts, der zu dem ersten, zweiten, dritten oder vierten Datensatz (12, 22, 32, 42) des Risikoindexes (i) gehört.

12. System (100) zum Berechnen eines Risikoindexes für eine Aortenruptur oder -dissektion bei einem Individuum mit thorakalem Aneurysma der Aorta ascendens (ATAA), wobei das System Folgendes umfasst:
erste Mittel (110) zum Erhalten eines ersten Datensatzes (12) in direktem Zusammenhang mit den klinischen und/oder demografischen Eigenschaften des Individuums; zweite Mittel (120) zum Erhalten eines Datensatzes (22) in direktem Zusammenhang mit den biochemischen Eigenschaften einer biologischen Probe des Individuums; dritte Mittel (130) zum Erhalten eines dritten Datensatzes (32) in direktem Zusammenhang mit den morphologischen und funktionellen Eigenschaften der Aorta; vierte Mittel (140) zum Erhalten eines vierten Datensatz (42), der durch die Verarbeitung des dritten Datensatzes (32) durch Modellrechnungen erhalten wird; und einen Computer (150), der eine Datenschnittstelle zum Erhalten des ersten Datensatzes (12), des zweiten Datensatzes (22), des dritten Datensatzes (32) und des vierten Datensatzes (42) als Eingabedaten und einen Prozessor zum Verarbeiten der Daten (12, 22, 32, 42) und zum Bereitstellen des Risikoindexes (i) für eine Aortenruptur oder -dissektion als Ausgabedaten aufweist, wobei der erste, der zweite, der dritte und der vierte Datensatz (12, 22, 32, 42) in einem Vorhersagemodell integriert werden, wobei der zweite Datensatz (22) Expressionswerte von mindestens einem Biomarker der nichtkodierenden RNA umfasst, ausgewählt aus der Gruppe, die folgende umfasst: miR-16, miR-9, miR-101, miR-143, miR-19, miR-21, miR-29 und miR-423-5p.

13. System nach Anspruch 12, wobei der zweite Datensatz (22) ferner Expressionswerte von mindestens einem Biomarker der nichtkodierenden RNA umfasst, ausgewählt aus der Gruppe, die folgende umfasst: miR-133a, miR-155, miR-320a, miR-34a (MI0001251), miR-34a (MI0000268) .

## Revendications

1. Procédé de calcul d'un indice de risque de rupture ou de dissection aortique d'un individu présentant un anévrisme de l'aorte thoracique ascendante, ATAA, le procédé comprenant les étapes suivantes :
obtention (10) d'un premier ensemble de données (12) lié aux caractéristiques cliniques et/ou démographiques de l'individu ;
obtention (20) d'un deuxième ensemble de données (22) lié aux caractéristiques biochimiques d'un échantillon biologique de l'individu ;
obtention (30) d'un troisième ensemble de données (32) lié aux caractéristiques morphologiques et fonctionnelles de l'aorte et traitement dudit troisième ensemble de données (32) pour obtenir (40) un quatrième ensemble de données (42) par modélisation informatique ; et
intégration du premier ensemble de données (12), du deuxième ensemble de données (22), du troisième ensemble de données (32) et du quatrième ensemble de données (42) dans un modèle prédictif pour obtenir un indice de risque (i) de rupture ou de dissection aortique ;
**caractérisé en ce que**
le deuxième ensemble de données (22) comprend des valeurs d'expression d'au moins un biomarqueur ARN non codant sélectionné dans le groupe comprenant : miR-16, miR-9, miR-101, miR-143, miR-19, miR-21, miR-29, et miR-423-5p.

2. Procédé selon la revendication 1, dans lequel le biomarqueur est sélectionné dans le groupe comprenant : une métalloprotéinase de la matrice extracellulaire, MMP, et un inhibiteur tissulaire, TIMP.

3. Procédé selon la revendication 1 ou 2, dans lequel le deuxième ensemble de données (22) comprend en outre des valeurs d'expression d'au moins un biomarqueur ARN non codant sélectionné dans le groupe comprenant : miR-133a, miR-155, miR-320a, miR-34a (MI0001251), miR-34a (MI0000268) .

4. Procédé selon la revendication 2 ou 3, dans lequel la métalloprotéinase de la matrice extracellulaire est la MMP-9 et l'inhibiteur tissulaire est TIMP-1.

5. Procédé selon l'une quelconque des revendications précédentes, dans lequel le biomarqueur est sélectionné dans le groupe comprenant : la protéine C réactive, la créatine kinase, Nt-proBNP, la troponine, un produit terminal de glycation avancée, AGE, et un récepteur correspondant, RAGE, le facteur de croissance transformant β, un D-dimère et l'interleukine 6, IL-6.

6. Procédé selon l'une des revendications précédentes, dans lequel le troisième ensemble de données (32) comprend des données morphologiques après une reconstruction virtuelle de l'anatomie de l'aorte de l'individu par un procédé d'imagerie diagnostique.

7. Procédé selon l'une des revendications précédentes, dans lequel le quatrième ensemble de données (42) comprend des paramètres hémodynamiques et structuraux de l'aorte estimés par une simulation numérique et dans lequel lesdits paramètres hémodynamiques et structuraux sont intégrés dans un modèle fluide-structure bidirectionnel.

8. Procédé selon les revendications 6 et 7, comprenant en outre un traitement des résultats de simulation numérique pour afficher les paramètres hémodynamiques et structuraux en les superposant sur la reconstruction virtuelle de l'anatomie aortique et en extrapolant lesdits paramètres dans différentes positions anatomiques de l'aorte.

9. Procédé selon la revendication 7 ou 8, dans lequel les paramètres hémodynamiques et structuraux comprennent au moins la pression artérielle, la contrainte de cisaillement, la contrainte intramurale et l'indice du flux hélicoïdal.

10. Procédé selon l'une des revendications précédentes, dans lequel le quatrième ensemble de données (42) comprend en outre des informations liées à une déformation de l'aorte et à une variation temporelle de ladite déformation obtenue par application d'un algorithme de suivi temporel.

11. Procédé selon l'une des revendications précédentes, comprenant en outre une évaluation de la pondération de chaque donnée appartenant aux premier, deuxième, troisième ou quatrième ensembles de données (12, 22, 32, 42) sur l'indice de risque (i).

12. Système (100) de calcul d'un indice de risque de rupture ou de dissection aortique d'un individu présentant un anévrisme de l'aorte thoracique ascendante, ATAA, le système comprenant :
un premier moyen (110) d'obtention d'un premier ensemble de données (12) lié aux caractéristiques cliniques et/ou démographiques de l'individu ;
un deuxième moyen (120) d'obtention d'un deuxième ensemble de données (22) lié aux caractéristiques biochimiques d'un échantillon biologique de l'individu ;
un troisième moyen (130) d'obtention d'un troisième ensemble de données (32) lié aux caractéristiques morphologiques et fonctionnelles de l'aorte ; un quatrième moyen (140) d'obtention d'un quatrième ensemble de données (42) obtenu à partir d'un traitement du troisième ensemble de données (32) au moyen d'une modélisation informatique ; et un ordinateur (150) ayant une interface de données pour recevoir le premier ensemble de données (12), le deuxième ensemble de données (22), le troisième ensemble de données (32) et le quatrième ensemble de données (42) sous la forme de données d'entrée et un processeur pour le traitement desdites données (12, 22, 32, 42) et délivrer en sortie l'indice de risque (i) de rupture ou de dissection aortique sous la forme de données de sortie, en intégrant le premier, le deuxième, le troisième et le quatrième ensemble de données (12, 22, 32, 42) dans un modèle prédictif, dans lequel le deuxième ensemble de données (22) comprend des valeurs d'expression d'au moins un biomarqueur ARN non codant sélectionné dans le groupe comprenant : miR-16, miR-9, miR-101, miR-143, miR-19, miR-21, miR-29, et miR-423-5p.

13. Système selon la revendication 12, dans lequel le deuxième ensemble de données (22) comprend en outre des valeurs d'expression d'au moins un biomarqueur ARN non codant sélectionné dans le groupe comprenant : miR-133a, miR-155, miR-320a, miR-34a (MI0001251), miR-34a (MI0000268) .
